# EUROPEAN PATENT SPECIFICATION

(11) **EP 1 458 432 B1**
(45) Date of publication and mention of the grant of the patent: **05.03.2008**
(21) Application number: 02788413.9
(22) Date of filing: 20.12.2002
(51) Int. Cl.: A61M 1/16, A61M 1/34, A61M 5/44, A61M 1/36

(54) **CONTROL EQUIPMENT IN AN EXTRACORPOREAL BLOOD CIRCUIT**
REGELEINRICHTUNG IN EINEM EXTRAKORPORALEN BLUTKREISLAUF
EQUIPEMENT DE COMMANDE DANS UN CIRCUIT SANGUIN EXTRACORPOREL

(30) Priority: 28.12.2001 IT MI20012829
(43) Date of publication of application: 22.09.2004
(73) Proprietor: Gambro Lundia AB, 22 643 Lund (SE)
(72) Inventor: FONTANAZZI, Francesco, I-41100 Modena (IT); ZACCARELLI, Massimo, I-41038 San Felice sul Panaro (IT); PAOLINI, Francesco, I-41010 Ganaceto (IT)
(74) Representative: Villanova, Massimo
(86) International application number: PCT/IB2002/005571
(87) International publication number: WO 2003/055543

(56) References cited:
- EP-A- 0 265 795
- EP-A- 1 132 101
- WO-A-00/41746
- US-A- 4 140 635
- US-A- 4 231 425

## Description

The present invention relates to **a blood-purifying machine having** control equipment for an extracorporeal blood circuit.

The extracorporeal circuit is generally connected to the patient by means of an access needle and a return needle, which are inserted into a fistula formed in the patient's cardiovascular system, and are used, respectively, to collect the blood to be treated via an access branch, and to return the treated blood to the patient's cardiovascular system via a return branch.

A first known process for purifying the blood comprises, in addition to the extracorporeal circuit for the circulation of the blood, a circuit for the preparation of a treatment liquid or a circuit for the circulation of dialysate solutions which are ready for use and are commonly called "dialysate", and a blood treatment element, which is commonly called a "filter", and is divided into two compartments by a semi-permeable membrane.

One of the compartments of the filter, called the "blood compartment", is connected to the extracorporeal circuit for the circulation of the blood and has the blood to be treated flowing through it during operation, while the other compartment of the filter has the dialysate flowing through it. The process of purifying the blood by means of a dialysate is called "haemodialysis".

Another blood purification process, known as "haemofiltration", is carried out by connecting the extracorporeal circuit to a filter, which is provided by a compartment through which the blood flows, and with a compartment acting as a receptacle for the undesired substances extracted from the blood.

A third process, which essentially combines the processes of haemodialysis and haemofiltration, is called haemodiafiltration.

During the blood purification treatment, the undesired particles contained in the blood migrate through the semi-permeable membrane from the blood compartment into the other compartment, either by convection (the phenomenon of convection is present in the process of haemofiltration, haemodialysis and haemodiafiltration), as a result of the passage of some of the blood liquid into the other compartment, or by diffusion (the phenomenon of diffusion is present in the processes of haemodialysis and haemodiafiltration), owing to the concentration gradient present between the blood and the dialysate.

Thus, at the end of the dialysis treatment, the patient will have lost some weight and the undesired substances will have been eliminated from the patient's blood.

The blood purification processes described above have variants which comprise the infusion of a replacement liquid into the extracorporeal circuit for the circulation of the blood, downstream of the filter (post-dilution) or upstream of the filter (pre-dilution).

In general, blood purification processes can be summarized as follows:
- the pure haemofiltration process, where no treatment fluid is used;
- the pre- or post-dilution haemofiltration process, where a replacement fluid is used upstream or downstream of the filter;
- the haemodialysis process, where the dialysate is used alone; and pre- or post-dilution haemodiafiltration processes, where both the dialysate liquid and the replacement liquid are used.

Given this general preliminary description, it should be noted that the blood extracted from the patient is normally at the temperature of 37°C and is conveyed along the extracorporeal circuit for the circulation of blood to enable the purification treatment to be carried out. During its travel along the extracorporeal circuit, the blood undergoes temperature variations due to the heat exchange with the surrounding environment and with the treatment fluids, when the blood purification process makes use of a treatment fluid. A widespread practice, associated with the processes which make use of a blood treatment fluid, is that of heating the dialysate and/or the replacement liquid, to prevent the patient from being brought into a state of hypothermia. However, it is extremely difficult to predict what the thermal equilibrium of the blood will be in the extracorporeal circuit, in order to determine the exact amount of heat to be supplied to the blood via the dialysate and/or the replacement liquid, and thus to re-establish the initial blood temperature.

Moreover, a number of reliable studies have shown that the blood purification treatment frequently causes a rise in the patient's blood temperature, due to the specific reaction of the blood to the materials used, or in other words to the incomplete biocompatibility of these materials with the patient's blood.

In general, it is exceedingly difficult to implement in a dialysis machine a method capable of precisely determining the thermal equilibrium of the blood and of compensating the temperature variations to which the patient is subject. This is because, in order to implement such a method, it is necessary to determine the blood temperature in a precise way by means of temperature sensors of the non-invasive type, whose accuracy is sometimes relatively low, to determine in a precise way the rate of flow of blood in the extracorporeal circuit, to determine the temperature and rate of flow of the dialysate and/or of the replacement liquid (when the blood purification process makes use of a treatment fluid), and to determine various heat exchange coefficients. In practice, the thermal equilibrium of the blood in the extracorporeal circuit can be established in the laboratory by using highly sophisticated instruments, but is difficult to achieve in blood purification machines.

The patent EP 265,795 discloses blood control equipment applied to a blood purification machine. This equipment withdraws heat from the blood or supplies heat to it in the extracorporeal circuit for the circulation of blood, by suitably controlling the temperature of the dialysate and/or replacement liquid, and as a function of the difference between the temperature of the blood leaving the patient and a predetermined temperature, or as a function of the difference between the temperature of the blood leaving the patient and the temperature of the blood in the return branch, and also as a function of the rate of flow of the blood in the extracorporeal circuit.

The equipment described in EP 265,795 has numerous drawbacks, of which the following appear to be most significant:
- studies have demonstrated, as reported in the text of EP 265,795, that a low temperature of the dialysate promotes the attainment of greater stability of the cardiovascular system, and consequently of the pressure of the patient, and reduces the occurrence of feverishness in the patient. However, according to EP 265,795 the blood temperature is clearly controlled in an indirect way, by heating the replacement liquid and/or the dialysate;
- the implementation of this control requires relatively complex equipment, and the drawing up of energy balances that are both accurate and complicated;
- unless it is adapted, the equipment described in EP 265,795 cannot regulate the temperature in a machine providing treatment with dialysate and also in a machine operating with a replacement liquid;
- the equipment cannot control the blood temperature in a machine providing a pure haemofiltration treatment.

EP1132101 shows a treatment apparatus where temperature of a body organ is regulated by controlling the temperature of an infusion fluid. The apparatus includes a blood concentration unit in an extracorporal blood circuit comprising a heat exchanger downstream of the blood concentration unit.

US6561997 shows a fluid circuit where heat exchangers are not operative downstream all treatment elements. Moreover none of the exchangers controls the temperature of blood as a function of a temperature detected upstream all treatment elements.

The object of the present invention is to provide control equipment for an extracorporeal blood circuit which overcomes the drawbacks of the known control equipment and which, in particular, is both efficient and easily implemented in all blood purification machines.

According to the present invention, **a machine is provided having the features of claim 1.**

The equipment according to the present invention makes it possible to dispense with the control of the temperature of the dialysate and/or replacement liquid. By suitably locating the regulation device within the return branch, it is possible to avoid the occurrence of phenomena which might further modify the blood temperature before the treated blood is returned to the patient. Furthermore, the control equipment interacts with the return branch and with the access branch only, and can be fitted to any blood purification machine.

To enable the present invention to be more clearly understood, a preferred embodiment thereof will now be described, purely by way of example and without restrictive intent, with reference to the attached figures, of which:
- Figure 1 is a schematic view, with parts removed for clarity, of a dialysis machine fitted with blood control equipment;
- Figure 2 is a schematic view of a haemofiltration machine fitted with the blood control equipment of Figure 1.

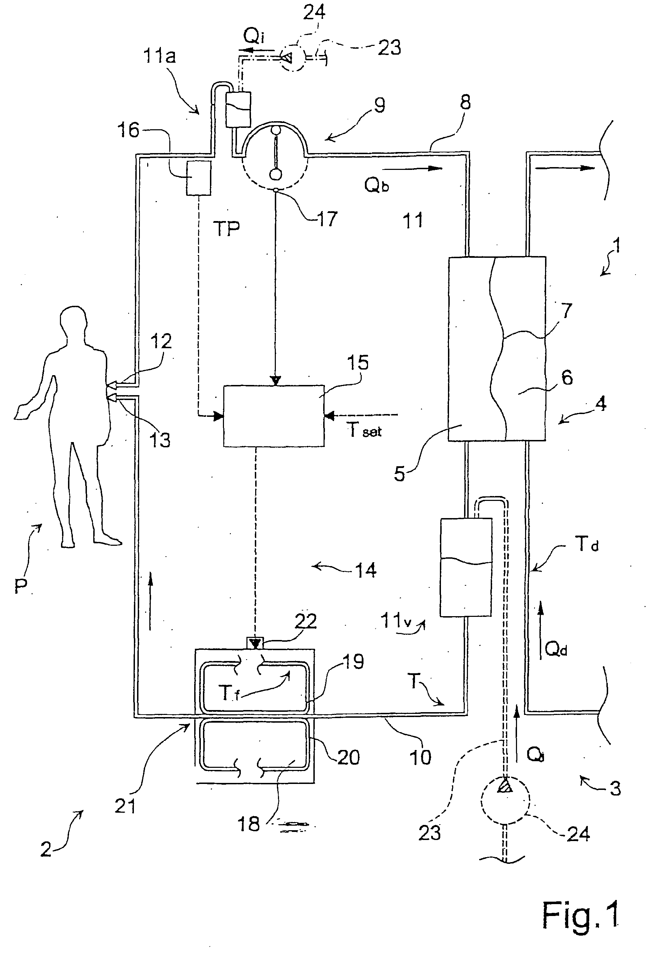

In Figure 1, the number 1 indicates the whole of a dialysis machine connected to a patient P. The machine 1 comprises an extracorporeal circuit 2 for the circulation of blood, a dialysate circuit 3 and a filter 4, which comprises a blood compartment 5 and a dialysate compartment 6 separated by a semi-permeable membrane 7.

The extracorporeal blood circuit 2 comprises an access branch 8, in which is located a peristaltic pump 9 providing a rate of blood flow Qb and an expansion chamber 11a upstream of the pump 9, and a return branch 10, in which an expansion chamber 11v is located. The access branch 8 has one end connected to the blood compartment 5 and one end provided with an access needle 12, which, during operation, is inserted into a fistula (not shown) in the patient P to collect the blood from the cardiovascular system of the patient P, while the return branch 10 has one end connected to the blood compartment 5 and an opposite end provided with a return needle 13, which, during operation, is inserted into the aforesaid fistula (not shown) to return the treated blood to the cardiovascular system of the patient P.

The machine 1 also comprises equipment 14 for regulating the blood temperature T in the extracorporeal circuit 2. The equipment 14 comprises a control unit 15 provided with a CPU, a temperature sensor 16 located in the access branch 8 upstream of the expansion chamber 11a, a sensor 17 to detect whether the peristaltic pump 9 is in operation, and a temperature regulator device 18 connected to a portion 19 of the return branch 10 downstream of the expansion chamber 11v, in such a way that it combines with the portion 19 to form a heat exchanger.

The device 18 regulates the blood temperature in the portion 19 without increasing the mass of the blood flow. In other words, the device 18 acts on a fluid which is physically separated from the blood and whose temperature Tf is controlled by the unit 15 in a range from 20°C to 43°C, in such a way that heat is supplied to or withdrawn from the blood circulating in the return branch 10 directly before the blood is returned to the patient P.

The device 18 comprises at least one line 20 which forms a series of windings or a tube bundle, and provides a seat 21 For housing the portion 19 of the return branch 10, and a heater/cooler 22 connected to the control unit 15.

In operation, during the dialysis treatment the blood is collected from the patient P and is conveyed along the extracorporeal circuit 2 at the flow rate Qb, while the dialysate is conveyed along the circuit 3 at a flow rate Qd. The sensor 16 measures the temperature TP and the control unit 15 operates the device 18, according to a predetermined algorithm, as a function of the temperature TP and of a reference temperature Tset which is set by an operator in the control unit 15.

For example, the control unit 15 compares the temperature TP with a reference temperature Tset, which is generally equal to 37°C, and calculates the temperature difference ΔT between the temperature TP and the reference temperature Tset. At the start of the dialysis treatment, the device 18 keeps the temperature Tf of the fluid at a value equal to the reference temperature Tset, while the temperature Td of the dialysate is regulated in such a way as to optimize the haemodialysis treatment. During the haemodialysis treatment, the blood temperature T along the extracorporeal circuit 2 varies as a result of heat exchange with the surrounding environment, with the dialysate, and with the fluid conveyed within the device 18, and as a function of the reaction of the patient P to the materials used in the blood treatment.

The temperature TP is measured by the sensor 16, for example at relatively short intervals during the dialysis treatment, and the unit 15 calculates the temperature difference ΔT at the same frequency as that of the measurement of the temperature TP. When the temperature difference ΔT between the temperature TP and the reference temperature Tset takes a negative value, the temperature Tf of the fluid is raised in such a way as to supply heat to the blood along the portion 19, while when the temperature difference ΔT takes a positive value the temperature Tf of the fluid is lowered in such a way as to withdraw heat from the blood along the portion 19. By repeating the procedure described above at short intervals of time, it is possible to rapidly stabilize the temperature TP, in other words the temperature of the patient P, at a value close to the reference temperature Tset, whenever there is a variation of the temperature TP with respect to the reference temperature Tset.

The sensor 17 detects the state of operation of the pump 9 and emits a signal to indicate when the pump 9 is operational and when it is stopped. If the signal emitted by the sensor 17 indicates that the pump 9 is in a stopped state, the control unit 15 keeps the value of Tf equal to the reference temperature Tset; if, on the other hand, the signal indicates that the pump 9 is in an operational state, the fluid temperature Tf is regulated as a function of the temperature difference ΔT according to the procedure described above.

In a variant of the operation, the reference temperature Tset is not fixed, but varies during the dialysis treatment according to a specified profile.

In a variant, the machine 1 is equipped with an infusion line shown in broken lines in Figure 1. The infusion line comprises an infusion branch 23 connected to the expansion chamber 11v of the return branch 10 and a pump 24 located in the branch 23 to provide a rate of flow Qi of replacement liquid which is introduced into the extracorporeal circuit 2. The replacement liquid can cause a further variation of the temperature T of the blood which is mixed with the replacement liquid.

The equipment 14 applied to the variant of Figure 1, and its mode of operation, are completely identical to those described with reference to the circuit of Figure 1 without the infusion process, although in the case of the variant the blood circulating in the extracorporeal circuit 2 is subjected to a first heat exchange in the blood compartment 5 of the filter 4 and to a second heat exchange in the expansion chamber 11v of the return branch 10. In this case, the heat generator 18 must be located downstream of the expansion chamber 11v of the return branch 10, to correct the variations of the blood temperature T before the blood is returned to the patient P.

In a further variant, the machine is equipped with an infusion line, which is shown in chained lines in Figure 1, and comprises the infusion branch 23 connected to the expansion chamber 11a of the access branch 8 and the pump 24 for providing the rate of flow Qi of the infusion liquid. In this case also, both the equipment 14 and the operation of the equipment 14 remain unaltered with respect to the cases described previously.

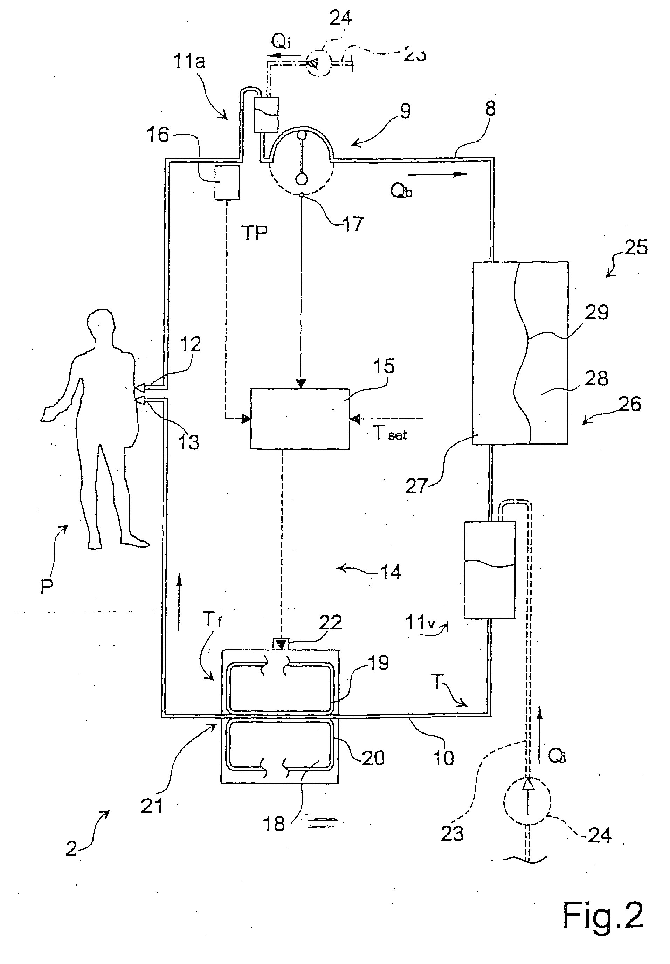

With reference to Figure 2, the number 25 indicates a haemofiltration machine, comprising the extracorporeal circuit 2 and a haemofiltration filter 26 comprising a blood compartment 27 and a compartment 28, separated by a semi-permeable membrane 29. The machine 25 is provided with blood control equipment 14, and also, in the variants illustrated in broken lines and in chained lines respectively, with a post-dilution and/or a pre-dilution infusion branch.

The machine 25 can carry out pure haemofiltration treatments and pre- and/or post-dilution haemofiltration treatments.

The equipment 14 applied to the machine 25, and its mode of operation, are completely identical to those associated with the machine 1.

The equipment 14 is particularly advantageous because it can be connected to any type of blood purification machine and does not require adaptation to the type of purification treatment which is administered.

## Claims

1. A blood purification machine (1; 25) comprising:
- one or more blood treatment elements (4; 4, 11a; 4, 11v; 25; 25, 11a; 25, 11v),
- an extracorporeal circuit (2) connected to the blood purification machine and comprising an access branch (8) and a return branch (10) connected to the at least one blood treatment element,
- a control equipment for the extracorporeal blood circuit (2), the equipment (14) comprising: a sensor (16) for measuring a first temperature (TP) of the blood leaving a patient (P) along the access branch (8) upstream of the said elements (4; 4, 11a; 4, 11v; 25; 25, 11a; 25, 11v), and a control unit (15) for regulating the blood temperature (T) as a function of the first temperature (TP) and of a reference temperature (Tset); wherein
the equipment comprises a device (18) for regulating the blood temperature (T) without increasing the mass of the blood flow by acting on a fluid which is physically separated from the blood, the device (18) being connected to a portion (19) of the return branch (10), downstream of the said blood treatment elements (4; 4, 11a; 4, 11v; 25; 25, 11a; 25, 1 1v),to form a heat exchanger; and
said control unit (15) is connected to the said temperature regulating device (18) and controls the temperature (Tf) of said fluid physically separated from blood as a function of the first temperature in a range from 20°C to 43°C, in such a way that heat is supplied to or withdrawn from the blood circulating in the return branch (10) directly before the blood is returned to the patient (P).

2. Machine according to Claim 1, **characterized in that** the reference temperature (Tset) is not fixed, but varies according to a specified profile.

3. Machine according to Claim 1, **characterized in that** the said regulating device (18) comprises a line (20) for conveying a fluid which can be heated to a temperature (Tf) lying within a specified range in the vicinity of a temperature of approximately 37°C.

4. Machine according to one of Claims lor 3, **characterized in that** the said regulating device (18) has a seat (21) for housing the said portion (19) of the return branch (10).

5. Machine according to one of Claims 1, 3 and 4, **characterized in that** the said extracorporeal circuit (2) is connected to a pump (9) for conveying the blood along the extracorporeal circuit (2), the equipment (14) comprising a sensor (17) for detecting the operating state of the pump (9); the control unit (15) keeping the temperature (Tf) of the said fluid equal to the said predetermined temperature (Tset) when the pump (9) is not in operation.

6. Machine according to one of Claims 1, and 3 to 5, **characterized in that** the said return branch (10) comprises an expansion chamber (11v); the said second portion (19) being located downstream of the expansion chamber (11).

7. Machine according to any one of Claims I and 3 to 6, **characterized in that** the said blood treatment element (4; 4, 11a; 4, 11v; 25; 25, 11a; 25, 11v) is formed by a haemodialysis filter (4) comprising a blood compartment (5) and a dialysate compartment (6), within which a dialysate flows.

8. Machine according to one of Claims 1, and 3 to 6, **characterized in that** the said blood treatment element (4; 4, 11a; 4, 11v; 25; 25, 11a; 25, 11v) comprises a haemodialysis filter (4) comprising a blood compartment (5) and a dialysate compartment (6), within which a dialysate flows, and an expansion chamber (11a; 11v), into which a replacement fluid is fed.

9. Machine according to one of Claims 1, and 3 to 6, **characterized in that** the said blood treatment element (4; 4, 11a; 4, 11v; 25; 25, 11a; 25, 11v) is formed by a haemofiltration filter (25).

10. Machine according to one of Claims 1, and 3 to 6, **characterized in that** the said blood treatment element (4; 4, 11a; 4, 11v; 25; 25, 11a; 25, 11v) comprises a haemofiltration filter (25) and an expansion chamber (11a; 11v), into which a replacement fluid is fed.

11. Machine according to Claim 1, **characterized in that** the said control unit (15) regulates the temperature (T) as a function of the first temperature (TP) and of the reference temperature (Tset) at predetermined intervals of time.

12. Machine according to Claim 1 or 11, **characterized in that** the said control unit (15) regulates the temperature (T) as a function of the difference between the first temperature (TP) and the reference temperature (Tset).

## Patentansprüche

1. Blutreinigungsmaschine (1; 25) umfassend.
- ein oder mehrere Blutbehandlungselemente (4; 4, 11a; 4, 11v; 25; 25, 11a; 25, 11v),
- einen extrakorporalen Kreislauf (2), der mit der Blutreinigungsmaschine verbunden ist und einen Zugangszweig (8) und einen Rückgabezweig (10), die mit dem mindestens einen Blutbehandlungselement verbunden sind, umfasst;
- ein Steuergerät für den extrakorporalen Blutkreislauf (2), wobei das Gerät (14): einen Sensor (16) zur Messung einer ersten Temperatur (TP) des einen Patienten (P) entlang dem Zugangszweig (8) aufwärts von den benannten Elementen (4; 4, 11a; 4, 11v; 25; 25, 11a; 25, 11v) verlassenden Blutes, und eine Steuereinheit (15) zur Einstellung der Bluttemperatur (T) abhängig von der ersten Temperatur (TP) und von einer Bezugstemperatur (Tset) umfasst; worin
das Gerät eine Vorrichtung (18) zur Einstellung der Bluttemparatur (T), ohne die Masse des Blutflusses zu steigern, durch Wirkung auf eine physikalisch vom Blut getrennte Flüssigkeit umfasst, wobei die Vorrichtung (18) mit einem Abschnitt (19) des Rückgabezweigs (10) abwärts von den benannten Blutbehandlungselementen (4; 4, 11a; 4, 11v; 25; 25, 11a; 25, 11v) verbunden ist, zur Bildung eines Wärmeaustauschers; und worin
die benannte Steuereinheit (15) mit der benannten Temperatureinstellungsvorrichtung (18) verbunden ist und die Temperatur (Tf) der benannten physikalisch vom Blut getrennten Flüssigkeit abhängig von der ersten Temperatur in einem Bereich von 20°C bis 43°C steuert, so daß Wärme zum bzw. vom im Rückgabezweig (10) zirkulierenden Blut zugeführt oder entnommen wird, gerade bevor das Blut zum Patienten (P) rückgegeben wird.

2. Maschine nach Anspruch 1, **dadurch gekennzeichnet, daß** die Bezugstemperatur (Tset) nicht fest ist aber sich gemäß einem definierten Profil ändert.

3. Maschine nach Anspruch 1, **dadurch gekennzeichnet, daß** die benannte Einstellungsvorrichtung (18) eine Leitung (20) zum Transportieren einer Flüssigkeit umfasst, die zu einer Temperatur (Tf) in einem definierten Bereich von ungefähr 37°C gewärmt werden kann.

4. Maschine nach einem der Ansprüche 1 oder 3, **dadurch gekennzeichnet, daß** die benannte Einstellungsvorrichtung (18) einen Sitz (21) zur Aufnahme des benannten Abschnitts (19) des Rückgabezweigs (10) besitzt.

5. Maschine nach einem der Ansprüche 1, 3 und 4, **dadurch gekennzeichnet, daß** der benannte extrakorporale Kreislauf (2) mit einer Pumpe (9) zum Transportieren des Blutes entlang dem extrakorporalen Kreislauf (2) verbunden ist, wobei das Gerät (14) einen Sensor (17) zur Erfassung des Betriebszustands der Pumpe (9) umfasst; wobei die Steuereinheit (15) die Temperatur (Tf) der benannten Flüssigkeit gleich wie die benannte vorgegebene Temperatur (Tset) aufrechterhält, wenn die Pumpe (9) nicht in Betrieb ist.

6. Maschine nach einem der Ansprüche 1 und 3 bis 5, **dadurch gekennzeichnet, daß** der benannte Rückgabezweig (10) eine Ausdehnungskammer (11v) umfasst; wobei der benannte zweite Abschnitt (19) abwärts von der Ausdehnungskammer (11) liegt.

7. Maschine nach einem der Ansprüche 1 und 3 bis 6, **dadurch gekennzeichnet, daß** das benannte Blutbehandlungselement (4; 4, 11a; 4, 11v; 25; 25, 11a; 25, 11v) aus einem Hämodialysefilter (4) besteht, der einen Blutabteil (5) und einen Dialysatabteil (6) umfasst, in dem ein Dialysat fließt.

8. Maschine nach einem der Ansprüche 1 und 3 bis 6, **dadurch gekennzeichnet, daß** das benannte Blutbehandlungselement (4; 4, 11a; 4, 11v; 25; 25, 11a; 25, 11v) einen Hämodialysefilter (4) umfasst, der einen Blutabteil (5) und einen Dialysatabteil (6), in dem ein Dialysat fließt, sowie eine Ausdehnungskammer (11a; 11v) umfasst, in die eine Substitutionsflüssigkeit zugeführt wird.

9. Maschine nach einem der Ansprüche 1 und 3 bis 6, **dadurch gekennzeichnet, daß** das benannte Blutbehandlungselement (4; 4, 11a; 4, 11v; 25; 25, 11a; 25, 11v) aus einem Hämofiltrationsfilter (25) besteht.

10. Maschine nach einem der Ansprüche 1 und 3 bis 6, **dadurch gekennzeichnet, daß** das benannte Blutbehandlungselement (4; 4, 11a; 4, 11v; 25; 25, 11a; 25, 11v) einen Hämofiltrationsfilter (25) und eine Ausdehnungskammer (11a; 11v) umfasst, in die eine Substitutionsflüssigkeit zugeführt wird.

11. Maschine nach Anspruch 1, **dadurch gekennzeichnet, daß** die benannte Steuereinheit (15) die Temperatur (T) abhängig von der ersten Temperatur (TP) und von der Bezugstemperatur (Tset) bei vorgegebenen Zeitintervallen einstellt.

12. Maschine nach Anspruch 1 oder 11, **dadurch gekennzeichnet, daß** die benannte Steuereinheit (15) die Temperatur (T) abhängig von dem Unterschied zwischen der ersten Temperatur (TP) und der Bezugstemperatur (Tset) einstellt.

## Revendications

1. Machine d'épuration du sang (1; 25) comprenant:
- un ou plusieurs éléments de traitement du sang (4; 4, 11a; 4, 11v; 25; 25, 11a; 25, 11v),
- un circuit extracorporel (2) relié à la machine d'épuration du sang et comprenant une ramification d'accès (8) et une ramification de retour (10) reliées à l'au moins un élément de traitement du sang,
- un appareil de commande pour le circuit extracorporel de sang (2), l'appareil (14) comprenant: un senseur (16) pour mesurer une première température (TP) du sang sortant du patient (P) le long de la ramification d'accès (8) en amont desdits éléments (4; 4, 11a; 4, 11v; 25; 25, 11a; 25, 11v), et une unité de commande (15) pour régler la température du sang (T) en fonction de la première température (TP) et d'une température de référence (Tset); où
l'appareil comprend un dispositif (18) pour régler la température du sang (T) sans augmenter la masse du flux sanguin, en agissant sur un fluide physiquement séparé du sang, le dispositif (18) étant relié à une portion (19) de la ramification de retour (10), en aval desdits éléments de traitement du sang (4; 4, 11a; 4, 11v; 25; 25, 11a; 25, 11v), pour former un échangeur de chaleur; et
ladite unité de commande (15) est reliée audit dispositif de réglage de la température (18) et commande la température (Tf) dudit fluide physiquement séparé du sang en fonction de la première température dans un intervalle de 20°C à 43°C, de sorte que de la chaleur soit introduite dans le ou prélevée du sang circulant dans la ramification de retour (10) juste avant que le sang n'est restitué au patient (P).

2. Machine selon la revendication 1, **caractérisée en ce que** la température de référence (Tset) n'est pas fixe mais varie selon un profilé défini.

3. Machine selon la revendication 1, **caractérisée en ce que** ledit dispositif de réglage (18) comprend une ligne (20) pour transporter un fluide qui peut être chauffé à une température (Tf) comprise dans un intervalle défini proche d'une température d'environ 37°C.

4. Machine selon l'une des revendications 1 ou 3, **caractérisée en ce que** ledit dispositif de réglage (18) est muni d'un siège (21) pour loger ladite portion (19) de la ramification de retour (10).

5. Machine selon l'une des revendications 1, 3 et 4, **caractérisée en ce que** ledit circuit extracorporel (2) est relié à une pompe (9) pour transporter le sang le long du circuit extracorporel (2), l'appareil (14) comprenant un senseur (17) pour détecter la condition opérationnelle de la pompe (9); l'unité de commande (15) maintenant la température (Tf) dudit fluide égale à ladite température préétablie (Tset) lorsque la pompe (9) n'est pas en fonction.

6. Machine selon l'une des revendications 1 et 3 à 5, **caractérisée en ce que** ladite ramification de retour (10) comprend une chambre de détente (11v); ladite deuxième portion (19) étant placée en aval de la chambre de détente (11v).

7. Machine selon l'une des revendications 1 et 3 à 6, **caractérisée en ce que** ledit élément de traitement du sang (4; 4, 11a; 4, 11v; 25; 25, 11a; 25, 11v) se compose d'un filtre d'hémodialyse (4) comprenant un compartiment sang (5) et un compartiment dialysat (6) dans lequel s'écoule un dialysat.

8. Machine selon l'une des revendications 1 et 3 à 6, **caractérisée en ce que** ledit élément de traitement du sang (4; 4, 11a; 4, 11v; 25; 25, 11a; 25, 11v) comprend un filtre d'hémodialyse (4) comprenant un compartiment sang (5) et un compartiment dialysat (6) dans lequel s'écoule un dialysat, et une chambre de détente (11a; 11v) dans laquelle est introduit un fluide de substitution.

9. Machine selon l'une des revendications 1 et 3 à 6, **caractérisée en ce que** ledit élément de traitement du sang (4; 4, 11a; 4, 11v; 25; 25, 11a; 25, 11v) se compose d'un filtre d'hémofiltration (25).

10. Machine selon l'une des revendications 1 et 3 à 6, **caractérisée en ce que** ledit élément de traitement du sang (4; 4, 11a; 4, 11v; 25; 25, 11a; 25, 11v) comprend un filtre d'hémofiltration (25) et une chambre de détente (11a: 11v) dans laquelle est introduit un fluide de substitution.

11. Machine selon la revendication 1, **caractérisée en ce que** ladite unité de commande (15) règle la température (T) en fonction de la première température (TP) et de la température de référence (Tset) à des intervalles de temps préétablis.

12. Machine selon la revendication 1 ou 11, **caractérisée en ce que** ladite unité de commande (15) règle la température (T) en fonction de la différence entre la première température (TP) et la température de référence (Tset).
